Europäisches Patentamt

European Patent Office

Office européen des brevets

(11) Publication number: **0 386 946**
A1

# EUROPEAN PATENT APPLICATION

(21) Application number: 90302205.1

(22) Date of filing: 01.03.90

(51) Int. Cl.⁵: **A61K 39/12, A61K 39/21,**
**A61K 39/215**

(30) Priority: 01.03.89 US 317380

(43) Date of publication of application:
**12.09.90 Bulletin 90/37**

(84) Designated Contracting States:
**AT BE CH DE DK ES FR GB GR IT LI LU NL SE**

(71) Applicant: SYNBIOTICS CORPORATION
**11011 Via Frontera**
**San Diego California 92127(US)**

(72) Inventor: **Beardsley, Terry Raymond**
**2377 North Iris Lane**
**Escondido, California 92026(IT)**

(74) Representative: **Sheard, Andrew Gregory et al**
**Kilburn & Strode 30, John Street**
**London WC1N 2DD(GB)**

(54) Feline infectious peritonitis Vaccine.

(57) A vaccine for animals and humans against infectious viruses for which humoral immunity generally is not protective is produced by infecting whole cells with live virus under conditions such that antigen expression takes place on the surface of the cells, then fixing and separating the infected cells.

## FELINE INFECTIOUS PERITONITIS VACCINE

The present invention relates to vaccines useful in the treatment of infectious viruses. More particularly, the invention relates to vaccines useful in the treatment of feline infectious peritonitis virus.

Feline infectious peritonitis (FIP), an acute to chronic viral disease of cats, is characterised by an insidious onset; persistent, non-responsive fever; perivascular, pyogranulomatous inflammatory responses, and a variety of additional signs depending on the tissue system affected. The mortality rate in clinical FIP is extremely high, approaching 100%. The causative virus is a member of the Coronaviridae family of viruses.

In addition to feline infectious peritonitis virus (FIPV), there are several antigenically related coronaviruses that infect the domestic cat as well as other members of the family Felidae. Some produce primary infections in the cat, while others produce primary infection in other animal species or humans but on occasion may also infect the cat.

To protect cats against FIP, several investigators have attempted to develop effective and safe vaccines by utilising various feline coronaviruses. With the exception of an occasional sublethal dose of virulent virus, all previously reported attempted immunisations of cats against FIPV have uniformly failed.

For example, Barlough et al in "Cats and Coronaviruses," Javma, 193, 796-800 (1988) states that a safe and effective feline coronavirus vaccine is not available and that attempts to produce a uniformly safe and effective broad spectrum vaccine have proven unsuccessful. Barlough speculates at p. 799 that the ideal vaccine would contain a FIPV strain capable of inducing cell mediated immunity without causing FIP.

Another researcher in the field, Niels C. Pederson, DVM, Ph.D. also reported attemptes at FIP immunisation. In one study kittens vaccinated with an avirulent biotype of the Black strain of FIP virus given oronasally developed both indirect fluorescent and virus neutralising antibodies, but were not protected against oronasal challenge exposure with virulent virus. In fact kittens vaccinated with avirulent virus were more readily infected when challenged than were non-vaccinated cats. The results of this study indicated that humoral immunity was not protective in FIPV infection. Immune serum from FIPV resistant cats also failed to passively protect susceptible animals against virulent virus given intraperitoneally or oronasally and actually sensitized them to infection. Based on the foregoing, Pederson concluded that humoral immunity is not protective by itself and may even be deleterious. In commenting on the nature of a natural protective immunity to FIP observed in some cats, Pederson speculated that this natural immunity may be largely cell mediated and if so, then future therapy should be directed at stimulating cell-mediated immunity, though he cautions that this will not be easy to accomplish. He also states that the results of studies with avirulent FIPV and sublethal doses of virulent FIPV indicate that only infection with virulent virus can induce protective immunity. (See Pederson et al, Am. J. Vet. Res., 44: 229-234 (1983) and 45: 2580-2585 (1984); Adv. Exp. Med. Biol., 173: 365-380 (1984).

A number of patents and patent publications have alleged successful attempts to immunise against FIP utilising attenuated strains of FIPV, e.g. U.S. Patent No. 4,571,380 and proteins and protein fragments derived from the nucleotide sequence and the amino acid sequence of FIPV Strain 79-1146, e.g. EP-A-246,979.

Scott, F., in "Immunisation of Cats Against Feline Infectious Peritonitis," Proceedings, 4th Annual Kal Kan Symposium, 3-8 (1988), discloses that he had tried several approaches to produce inactivated FIPV vaccine using virus from cell cultures. Infectious virus was inactivated with formalin, beta-propiolactone or other inactivants, but was unable to demonstrate any protection against virulent virus challenge in cats vaccinated with the inactivated virus. He concluded that to date, attempts to produce an inactivated whole virus vaccine have been unsuccessful.

FIPV Strain 79-1146 (originally designated as WSU FIPV 79-1146) is a virus strain originally isolated by Dr. James F. Evermann, Washington State University and has been characterised by McKeirnan et al, Feline Practice, 11: 16-20 (1981).

The Applicants have now discovered a safe and effective vaccine which may be used to immunise animals, including humans, against a virus to which humoral immunity generally is not protective.

Thus according to a first aspect of the present invention there is provided a vaccine (suitable for immunising a human or animal) against a virus to which humoral immunity is generally not protective, comprising an inoculant which provides substantial cell mediated immunity against a virus, but without providing substantial humoral immunity against the virus.

The virus is suitably a coronavirus, such as FIPV, FECV, TGEV or canine coronavirus, or a retrovirus such as HIV, FIV, HTLV-I or HTLV-II.

The vaccine preferably comprises, as the inoculant, fixed (whole) cells that have been infected or transfected with the live virus, so that viral antigen expression occurs on the cell surface. Fixing can be achieved (after antigen expression) by a chemical fixing agent such as glutaraldehyde.

Preferred cells are Felis Catus Whole Fetus cells or Crandall Feline Kidney cells. The vaccine is suitable for both animals and humans, and in particular feline animals, especially where the virus is FIPV, as the FIPV strain 79-1146.

The vaccine can be prepared by first infecting suitable whole cells with live virus and then after an incubation period during which viral antigen expression takes place on the cell surface, the cells are fixed. After fixing, the fixed, whole cells are separated. Inoculation of an animal with these fixed whole cells provides a substantial cell mediated immunity against the virus without providing substantial humoral immunity against the virus. In this manner, a human or animal may be safely and effectively immunised against the virus.

A preferred process for the preparation of the innoculant, such as the fixed whole cells, comprises:

(a) infecting or transfecting suitable whole cells with the live virus;

(b) incubating the cells under conditions that antigen expression takes place on the surface of the cells; and

(c) fixing the resulting cells. The process preferably includes: (d) separating the fixed cells.

Thus a particularly preferred process is a method of producing a vaccine for protecting felines against feline infectious peritonitis virus (FIPV) caused infection which comprises:

(a) infecting feline derived whole cells with live FIPV;

(b) incubating the, e.g. infected, cells under conditions such that antigen expression takes place on the surface of the cells;

(c) fixing the resulting cells; and

(d) preferably separating the fixed cells, and the vaccine resulting therefrom.

Thus the present invention also relates to a vaccine for protecting a human or animal against an infectious virus comprising suitable fixed whole cells having the corresponding viral antigen expressed on the surface of the cells.

The term "suitable", as used herein in reference to the phrase "suitable fixed whole cells", is intended to mean cells which may be advantageously used with the species to be protected and which will not be rejected by the immune system of the host. For example, a suitable cell for a cat is a feline derived cell e.g., Felis Catus Whole Fetal cells or Crandall Feline Kidney cells.

A. Virus

The viruses to which this invention applies includes viruses to which humoral immunity generally is not protective, e.g., coronaviruses, such as, for example, FIPV, Feline Enteric Coronavirus (FECV), Transmissable Gastroenteritis Virus (TGEV) and canine coronaviruses, and retroviruses, such as, for example, Human Immunodeficiency Virus (HIV), Feline Immunodeficiency Virus (FIV), and other immunodeficiency viruses (e.g. HTLV-I and HTLV-II) and other envelope viruses.

The preferred Feline infectious peritonitis virus (FIPV) Strain 79-1146 originally designated as WSU FIPV 79-1146, was originally isolated by Dr. J.F. Evermann at Washington State University. Other suitable strains of FIPV may also be used.

B. Cells

The preferred whole cells will vary depending on whether a human, or an animal and the species of animal, is to be treated. For example, for cats, suitable cells lines are Felis Catus Whole Fetus (fcwf) cells, a continuous cell line originally described as a macrophage-like cell line by Pederson, et al, Am. J. Vet. Res. 42: 363 (1981). Other suitable feline whole cells may also be used, e.g., Crandall Feline Kidney cells. Comparable cell lines may be selected by one of ordinary skill in the art of each species to be treated.

C. Infection of Transfection

If the whole cells are to be infected, confluent cultures of cells are washed to remove media and then infected with live virus; or for transfection, transfected with recombinant DNA derived from the virus (see, for example, Graham et al, Virology 52:456-461). Suitably after washing to remove free virus, media may be added back to the culture which is then incubated under conditions such that antigen expression takes place on the surface of the cells. That is to say, the infected cells are suitably incubated for a time and at a temperature which will promote expression of viral antigen on the surface of the (infected) cells, but which will not allow the production of free virus or free antigen by the (infected) cell. The time and temperature of incubation will vary with the virus and the (infected) cell, but can be determined without undue experimentation. This can be done by visual inspection and by analysis of cells stained with viral specific antibodies coupled to fluorescein using a flow cytometer. For example, a suitable time and temperature of incubation of FIPV with fcwf cells is about 6 hours at $37^{\circ}$ C (incubation in excess of 8 hours allows release of virus and causes destruction of the cells). Cells may then be washed extensively to remove any remaining extracellular virus and serum proteins. Cells can then be separated by conventional means for fixation.

D. Fixation

The (infected) cells may then be counted, diluted to the desired concentration, preferably about $5 \times 10^6$ cells/ml, and then fixed. The fixing is done preferably with a chemical fixing agent, such as, for example, an alcohol, aldehyde or ketone. Suitable fixing agents include, but are not limited to, formaldehyde, glutaraldehyde, and other conventional fixing agents, see, for example, Clark, Ed., Staining Procedures, 4th Edition, 1981, Williams & Wilkins, publisher and Cell Biology in Medicine, E. Biltar, Ed., John Wiley & Sons, 1973. The preferred chemical fixing agent is glutaraldehyde, suitably in a concentration ranging from 0.01 to 1% and preferably from 0.1 to 0.5% and advantageously 0.25%, e.g. in conventional buffered saline solution. The preferred buffer is phosphate buffer. The time for fixing varies with the fixing agent and the concentration of the fixing agent and ranges from 5 to 60 minutes. When using the preferred buffer 0.25% glutaraldehyde in phosphate buffer, a preferred fixation time is about 15 minutes. The cells are suitably then washed, preferably three times in buffered saline and preferably treated with L-lysine to block non-specific binding of serum antibody to the cells through charge neutralisation.

Each batch of fixed cells so produced is suitably tested for sterility, potency, toxicity and the like according to suitable standards, e.g., guidelines laid down by the British Pharmacopoeia (Veterinary) 1977 page 149. The fixed cells may then conveniently be frozen in ampoules or vials, and stored in liquid nitrogen. Alternatively stabilisers such as sorbitol and/or protein hydrolysate, e.g. Sol-U-pro, may be added to enable the preparation to be freeze-dried.

A second aspect of the present invention relates to a pharmaceutical or veterinary composition, suitable for protecting a human, or an animal, e.g. feline animal, against an infectious virus, the composition comprising suitable whole fixed cells, as described in the first aspect, and a pharmaceutically or veterinarily acceptable carrier.

Thus in a preferred embodiment of the invention, there is provided a vaccine (e.g. for developing immunity in animals to FIPV) which comprises (suitable whole) fixed cells, as hereinbefore defined, in an effective dosage, or multiples thereof, in a pharmaceutically or veterinarily acceptable carrier. The effective dosage for vaccination may be from about $10^5$ cells to about $10^9$ cells and preferably about $3 \times 10^6$ cells to about $3 \times 10^8$ cells or equivalent cellular material.

The pharmaceutically or veterinarily acceptable carrier for the vaccine can be a liquid, such as an aqueous solution containing nutrients and stabilisers, e.g., Hank's balanced salt solution or other similar media. The carrier may, in some instances, include a sterile sealed container, such as an ampoule or vial.

The vaccine of the present invention can be administered to humans or animals desirably by intraperitoneal, subcutaneous, or intramuscular injection or via the oral, nasal or intraocular route. The dose may be administered as a single dose or as a multiplicity of sub-doses over a period of time. The preferred schedule is to administer a single dose of 1-2 ml containing the effective dosage of fixed cells.

According to a third aspect of the present invention there is provided an inoculant, such as suitable fixed whole cells, or a vaccine, as described in the first aspect, for use in human or veterinary medicine.

A fourth aspect of the present invention relates to the use of an inoculant, such as suitable fixed whole cells, as described in the first aspect, in the preparation of a vaccine.

Thus in a preferred embodiment there is provided the use of fixed whole cells having an antigen of a virus expressed on the surface of the cells in the preparation of a vaccine against the (infectious) virus.

A fifth aspect of the present invention encompasses a process for the preparation of a vaccine, the process comprising admixing an inoculant, such as suitable whole fixed cells, as previously described in the first aspect, with a pharmaceutically or veterinarily acceptable carrier.

A sixth aspect of the present invention relates to a product comprising an inoculant, such as suitable whole fixed cells, as described in the first aspect, and a vaccine, such as disease inducing antigenic

4

material, as a combined preparation for simultaneous, separate or sequential use in vaccination.

A particularly preferred embodiment of the present invention relates to a process for the preparation of an inoculant or a vaccine for protecting felines against feline infectious peritonitis viris (FIPV) caused infection, the process comprising:

(a) infecting or transfecting whole cells with live FIPV;

(b) incubating the infected (or transfected) cells under conditions such that antigenic expression takes place on the surface of the cells;

(c) fixing the resulting cells;

(d) optionally separating the fixed cells. The cells are preferably Felis Catus whole fetus cells.

Thus the vaccine of the present invention may also be combined with other vaccines comprising antigenic material derived from other microorganisms that cause disease, e.g., in members of the genus Felix, such as parvovirus, e.g., feline panleucopenia (feline infectious enteritis), feline calicivirus, feline rabies, feline retrovirus, e.g. feline leukaemia virus, and feline herpes viruses. A particularly preferred combination, presented as a vaccine pack, comprises the (suitable whole) fixed cell preparation in a sterile vessel together with a vessel containing a freeze-dried preparation of one or more other, e.g. feline, vaccines, suitably adapted to be combined before usage.

Alternatively the (suitable whole) fixed cell preparation may be presented as a freeze-dried preparation, with the other vaccine(s) being presented in wet form. In yet a further combination the fixed cell preparation may be freeze-dried in combination with one or more of the above mentioned vaccines and presented together with a vessel containing sterile water for use in reconstituting the freeze-dried vaccine prior to administration. The vessels may be packaged in a box or container together with instructions for use, which include the instruction to reconstitute the freeze-dried vaccine with the wet preparation or sterile water prior to administration.

It will be appreciated that preferred features and characteristics of the second to sixth aspects of the present invention are as for the first, mutatis mutandis.

The invention will now be described by way of example, with reference to the accompanying Examples, which are to be interpreted as being merely illustrative, but not limiting.

## EXAMPLE 1

Preparation of a Vaccine Against Feline Infectious Peritonitis Virus (FIVP).

Felis Catus Whole Fetal (fcwf) cells were trypsinised and seeded at $10^6$ cells per plate. The FIPV inoculum contained $1.75 \times 10^5$ plaque forming units (pfu)/ml. Selected plates were given 0.02-0.1 multiplicity of infection (moi) units of virus for 2 hours. It was determined that 0.02 moi was optimal for maximal viral antigen expression and minimal whole virus production. The infected cells were incubated at 37°C for 6 hours. Cells were adjusted to $2 \times 10^6$ cell/ml in phosphate buffered saline (PBS). Glutaraldehyde (GH) was added slowly with gentle stirring to a final concentration of 0.5% (volume/volume), and cells were fixed for 10 minutes at room temperature (RT). Cells were then washed twice with phosphate buffer solution (PBS). A final incubation was done for 2 hours at RT with 0.2M lysine at $2 \times 10^6$ cells/ml for absorption of residual GH on fixed cells. Cells were washed finally in PBS, and adjusted to $30 \times 10^6$ cells.ml in PBS for injection into cats.

## COMPARATIVE EXAMPLE 2

Vaccination of Cats Against FIPV.

Specific pathogen free (SPF) cats were inoculated intraperitoneally (ip) with $30 \times 10^6$ cells (from Example 1) on days 0, 14, and 28. Animals were challenged 10 days after the final immunisation with $1 \times 10^5$ PFU of FIPV 79-1146 Strain. In one study, 2 of 3 cats survived significantly longer than control unimmunised cats and 1 cat was a long term survivor. In a second study 3 of 6 cats survived significantly longer than controls and 2 cats were long term survivors. The results of the two studies are shown in the Table below.

EP 0 386 946 A1

|  | Survivors Ten Weeks Post FIPV Challenge |
|---|---|
| Control Cats | 1/7 |
| Vaccinated Cats | 5/9 |

**Claims**

1. A vaccine for immunising a human or an animal against a virus to which humoral immunity generally is not protective, the vaccine comprising an inoculant which provides substantial cell mediated immunity against the virus without providing substantial humoral immunity against the virus.

2. A vaccine as claimed in Claim 1 wherein the virus is a coronavirus such as FIPV, FECV, TGEV or canine coronavirus, or a retrovirus such as HIV, FIV, HTLV-I or HTLV-II.

3. A vaccine as claimed in Claim 1 or 2 wherein the inoculant comprises fixed cells previously infected or transfected with the live virus, optionally fixed with a chemical fixing agent such as glutaraldehyde.

4. A vaccine as claimed in Claim 3 wherein the cells are Felis Catus Whole Fetus cells or Crandall Feline Kidney Cells and where the virus is preferably FIPV, such as FIPV Strain 79-1146.

5. Suitable whole fixed cells having a viral antigen expressed on the surface of the cells for use in human or veterinary medicine.

6. The use of an inoculant which provides substantial cell mediated immunity against a virus without providing substantial humoral immunity against the virus in the preparation of a vaccine for an animal, such as a feline animal or a human against the virus to which humoral immunity generally is not protective.

7. The use as claimed in claim 6 wherein the virus is a coronavirus such as FIPV, FECV, TGEV or canine coronavirus, or a retrovirus such as HIV, FIV, HTLV-I and HTLV-II.

8. The use as claimed in Claim 6 or 7 wherein the inoculant comprises fixed cells, previously infected or transfected with the live virus, optionally fixed with a chemical fixing agent such as glutaraldehyde.

9. A process for the preparation of a vaccine comprising fixed whole cells, the process comprising:

(a) infecting or transfecting whole cells with a live virus;

(b) incubating the infected or transfected cells under conditions such that antigen expression takes place on the surface of the cells;

(c) fixing the resulting cells;

(d) optionally separating the fixed cells.

10. A process as claimed in Claim 9 wherein the cells are Felis Catus Whole Fetus cells, the virus is FIPV such as FIPV Strain 79-1146, and/or the cells are fixed with a chemical fixing agent such as glutaraldehyde.

11. A pharmaceutical or veterinary composition comprising suitable fixed whole cells having a viral antigen expressed on the surface of the cells and a pharmaceutically or veterinarily acceptable carrier.

12. A composition as claimed in Claim 11 wherein the virus is feline infectious peritonitis virus (FIPV) and the fixed whole cells have FIPV antigen expressed on the surface of the cells.

13. A composition as claimed in Claim 11 or 12 wherein the cells are Felis Catus Whole Fetus cells, and/or the FIPV is FIPV Strain 79-1146.

14. A product comprising suitable whole fixed cells having viral antigen expressed on the surface of the cells, and a vaccine, as a combined preparation for simultaneous, separate or sequential use in vaccination.

Claims for the following Contracting States : ES, GR

1. The use of an inoculant which provides substantial cell mediated immunity against a virus without providing substantial humoral immunity against the virus in the preparation of a vaccine for an animal, such as a feline animal or or a human against the virus to which humoral immunity generally is not protective.

2. The use as claimed in claim 6 wherein the virus is a coronavirus such as FIPV, FECV, TGEV or canine coronavirus, or a retrovirus such as HIV, FIV, HTLV-I and HTLV-II.

3. The use as claimed in Claim 6 or 7 wherein the inoculant comprises suitable whole fixed cells, previously infected or transfected with the live virus, optionally fixed with a chemical fixing agent such as glutaraldehyde.

4. The use as claimed in any of Claims 1 to 3 wherein the inoculant comprises suitable whole fixed

6

cells having the corresponding viral antigen expressed on the surface of the cells.

5. The use as claimed in Claim 3 wherein the cells are Felis Catus Whole Fetus cells or Crandall Feline Kidney cells.

6. A process for the preparation of a vaccine, the process comprising admixing an inoculant which provides substantial cell mediated immunity against a virus without providing substantial humoral immunity against the virus, with a pharmaceutically or veterinarily acceptable carrier.

7. A process as claimed in Claim 7 wherein the inoculant comprises suitable whole fixed cells having the corresponding viral antigen expressed on the surface of the cells.

8. A process as claimed in Claim 6 or 7 wherein the virus is a coronavirus such as FIPV, FECV, TGEV or canine coronavirus, or a retrovirus such as HIV, FIV, HTLV-I or HTLV-II.

9. A process as claimed in Claim 7 or 8 wherein the suitable whole fixed cells are prepared by:

(a) infecting or transfecting whole cells with live virus;

(b) incubating the infected or transfected cells under conditions such that antigen expression takes place on the surface of the cells;

(c) fixing the resulting cells;

(d) separating the fixed cells.

10. A process as claimed in any of Claims 7 to 9 wherein the cells are Felus Catus Whole Fetus cells or Crandall Feline Kidney cells.

11. A process as claimed in any of claims 6 to 10 wherein the virus is FIVP Strain 79-1146.

12. A process as claimed in Claim 10 or 11 wherein the cells are fixed with a chemical fixing agent such as glutaraldehyde.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 011 865 (THE WELLCOME FOUNDATION LTD) ----- | | A 61 K 39/12<br>A 61 K 39/21<br>A 61 K 39/215 |
| | | | TECHNICAL FIELDS SEARCHED (Int. Cl.5) |
| | | | A 61 K |

The present search report has been drawn up for all claims

| Place of search | Date of completion of the search | Examiner |
|---|---|---|
| THE HAGUE | 19-06-1990 | REMPP G.L.E. |